# EUROPEAN PATENT APPLICATION

(11) **EP 4 652 938 A1**
(43) Date of publication of application: **26.11.2025**
(21) Application number: 24177247.4
(22) Date of filing: 22.05.2024
(51) Int. Cl.: A61B 8/08, A61B 8/06

(54) **EVALUATING CARDIAC PARAMETERS**

(71) Applicant: Koninklijke Philips N.V., 5656 AG Eindhoven (NL)
(72) Inventor: GOSHEN, Elad, Eindhoven (NL); KALISMAN, Oren, Eindhoven (NL); YAACOBI, Marina, 5656AG Eindhoven (NL)
(74) Representative: Philips Intellectual Property & Standards

(57) **Abstract**

Proposed concepts thus aim to provide schemes, solutions, concepts, designs, methods and systems pertaining to a method for determining a value of a cardiac parameter of a cardiovascular system based on a plurality of ultrasound images of the cardiovascular system. The method involves utilizing information about the view of each of the plurality of ultrasound images and an associated view identification probability describing the likelihood that the view of a given ultrasound image has been identified correctly.

## Description

### FIELD OF THE INVENTION

This invention relates to the field of cardiovascular systems, and in particular to determining the value of cardiac parameters of cardiovascular systems.

### BACKGROUND OF THE INVENTION

There may be many different parameters of cardiovascular systems that are important for the diagnosis and assessment of patients. Determining the value of such parameters accurately and reliably may therefore be of great value.

Echocardiography is an important imaging technique for clinical diagnosis of heart diseases. Due to availability, low costs and its non-invasive nature, echocardiography is the most widely used imaging modality for cardiac function evaluation allowing for qualitative and quantitative evaluation of the heart. Global left ventricular (LV) function evaluation is often performed by clinicians during an echocardiogram. This may involve the calculation of the left ventricular Ejection Fraction (LV EF) and the Strain, which may be used to identify specific abnormalities or disfunctions of the cardiac system. Historically, the evaluation of these parameters is done by manual assessment of ultrasound images by a health care professional or by using semi-automated or automated software developed by different manufacturers. This may often lead to inconsistencies between practitioners and between the systems of different manufacturers.

Further, it is usual for many different ultrasound images to be taken during one examination of a patient. These may have different views and be of different quality. This may lead to further inconsistencies in the evaluation of cardiac parameters as the use of different images may result in different values for the parameter.

### SUMMARY OF THE INVENTION

The invention is defined by the claims.

According to examples in accordance with an aspect of the invention, there is provided a computer-implemented method for determining a value of a cardiac parameter of a cardiovascular system based on a plurality of ultrasound images of the cardiovascular system.

The computer-implemented method comprises: processing the plurality of ultrasound images with a view identification model to determine a view of each of the plurality of ultrasound images and an associated view identification probability describing the likelihood the determined view of the ultrasound image is correct; determining, based on the plurality of ultrasound images and the determined view of each of the plurality of ultrasound images, a plurality of ultrasound images sets each comprising one or more ultrasound images of the plurality of ultrasound images; determining for each of the plurality of image sets, based on the view identification probabilities of the one or more ultrasound images of each of the plurality of ultrasound image sets, a prediction value describing a predicted usefulness of the ultrasound image set for determining a value of the cardiac parameter of the cardiovascular system; and determining, based on the plurality of ultrasound image sets, a value of the cardiac parameter of the cardiovascular system.

In particular, embodiments aim to provide an efficient and standardized method by which a cardiac parameter of a cardiovascular system can be determined from a plurality of ultrasound images by determining the view of each of the plurality of ultrasound images and sorting the images into different sets of images based on the determined view. The probability of the view of each image having been identified correctly is then further used to predict the potential usefulness of the different image sets for evaluating the cardiac parameter.

Quantitative evaluation of cardiac parameters is routinely done via manual, semi-automated, or fully automated tools during ultrasound imaging examinations of cardiovascular systems. However, conventional approaches for achieving this are specific to different ultrasound image machines and their methodologies can vary significantly. Therefore, for Echo labs using devices and workstations of multiple vendors, there are often significant variations in the determined values of the same cardiac parameter. The proposed concept of the present invention allows for post processing of ultrasound images that may be integrated into any known or novel ultrasound imaging system and thereby provides a standardized method for determining the value of a cardiac parameter.

During an ultrasound imaging examination multiple images of the same cardiovascular system are taken using different system settings and different view orientations. Different cardiac parameters may be optimally calculated from different views. In proposed embodiments, the view of each ultrasound image is determined and used to organize the plurality of ultrasound images obtained during an ultrasound imaging examination of a patient into sets of images suitable for determining the value of the cardiac parameter. Embodiments may therefore be advantageous by incorporating view identification into the calculation of the cardiac parameter, allowing for a reliable and standardized measurement of the cardiac parameter to be obtained from the plurality of ultrasound images.

It may not be obvious which images of the plurality of ultrasound images should be used for cardiac parameter evaluation as there may be multiple images with the required view. The inventors of this application realized that the probability that the views of each image have been identified correctly may be used to predict the usefulness of different sets of images of the plurality of ultrasound images for determining the value of the cardiac parameter. Therefore, the proposed invention is advantageous in that it leverages the view identification probabilities in the determination of which images of the plurality of ultrasound images to use for cardiac parameter evaluation, resulting in a more efficient and accurate calculation.

In summary, it is proposed to leverage automatic view detection and associated view identification probabilities to sort images into image sets suitable for determining the value of the cardiac parameter. A prediction of how useful each image set will be for the determination of the value of the cardiac parameter can then be estimated and used to determine the value of the cardiac parameter. In this way, the proposed method has improved efficiency and reliability.

Ultimately, an improved method/system for automatically determining the value of a cardiac parameter of a cardiovascular system from ultrasound images of the cardiovascular system may be supported by the proposed concept(s).

In some embodiments, the plurality of ultrasound image sets may comprise a plurality of ultrasound image triplets, each of the plurality of ultrasound image triplets comprising: a first image being an ultrasound image of the plurality of ultrasound images determined to have a two-chamber apical (2ch) view; a second image being an ultrasound image of the plurality of ultrasound images determined to have a three-chamber apical (3ch) view; and a third image, being an ultrasound image of the plurality of ultrasound images determined to have a four-chamber apical (4ch) view. In this way, the proposed method may be configured to allow for the accurate determination of the left ventricular ejection fraction and the average global longitudinal strain for which the 2ch, 3ch, and 4ch views are important.

In some embodiments, the plurality of ultrasound image sets may comprise a plurality of ultrasound image pairs, each of the plurality of ultrasound image pairs comprising: a first image being an ultrasound image of the plurality of ultrasound images determined to have a two-chamber apical (2ch) view; and a second image being an ultrasound image of the plurality of ultrasound images determined to have a four-chamber apical (4ch) view. The 2ch and 4ch views may be less optimal than a full image triplet but may still allow for calculation of the EF and a strain parameter.

In some embodiments, determining the value of a left ventricular parameter of the cardiac system may comprise: determining an ordered list of the plurality of ultrasound image sets based on the determined prediction value of each ultrasound image set; processing the first ultrasound image set of the ordered list with a cardiac parameter determination model; and processing the second ultrasound image set of the ordered list with the cardiac parameter determination model if processing the first ultrasound image set of the ordered list does not result in the determination of the value of the cardiac parameter of the cardiovascular system. This may provide an improved efficiency in determining the value of the cardiac parameter of the cardiovascular system as only the image set most likely to produce a value of the cardiac parameter is processed.

In some embodiments, determining the plurality of ultrasound image sets may comprise: comparing the view identification probability of each of the plurality of ultrasound images to a threshold probability value; and disregarding a set of ultrasound images of the plurality of ultrasound images based on this comparison. Filtering the ultrasound images based on the determined view identification probabilities allows for only images of sufficient quality for analysis to be used for the determination of the cardiac parameter, increasing the likelihood that automated analysis will result in a value of the cardiac parameter being determined and reducing the subjectivity of any calculated value.

In some embodiments, the threshold probability value may be in the range 0.7 to 1, and preferably wherein the threshold probability value is in the range 0.8 to 0.9. These ranges have been shown to allow for efficient filtering of the plurality of ultrasound images.

In some embodiments, the plurality of ultrasound images comprises depth data comprising a depth value of each of the plurality of ultrasound images, and determining the plurality of ultrasound image sets may comprise: comparing the depth value of each of the plurality of ultrasound image to a threshold depth value; and disregarding a set of ultrasound images based on this comparison. This may improve the efficiency of the method as images that correspond to depths that are known to not allow the accurate determination of cardiac parameters can be automatically discarded.

In some embodiments, the threshold depth value may be in the range 8 cm to 12 cm, and disregarding a set of ultrasound images of the plurality of ultrasound images may comprise disregarding the set of ultrasound images with depth values less than the threshold depth value. Introducing a minimum threshold depth value in this way may improve the accuracy of the determined value of the cardiac parameter.

In some embodiments, the threshold depth value may be in the range 20 cm to 24 cm, and disregarding a set of ultrasound images of the plurality of ultrasound images comprises disregarding the set of ultrasound images with depth values greater than the threshold depth value. Introducing a maximum threshold depth value in this way may improve the accuracy of the determined value of the cardiac parameter.

In some embodiments, the plurality of ultrasound images comprises depth data, the depth data comprising a depth value of each of the plurality of ultrasound images. Each of the plurality of ultrasound image sets may comprise two or more ultrasound images of the plurality of ultrasound images, and determining the plurality of ultrasound image sets may comprise: determining, based on the plurality of ultrasound images and the determined views of each of the plurality of ultrasound images, a plurality of set candidates each set candidate comprising two or more candidate images of the plurality of ultrasound images; comparing the depth values of the two or more candidate images of each of the plurality of set candidates; and disregarding a portion of set candidates of the plurality of set candidates based on this comparison to determine the plurality of ultrasound image sets. Image sets that contain images of very different depths are unlikely to result in a reliable determination of the value of the cardiac parameter. Therefore, by filtering out image sets based on depth values after candidate sets have been formed, the proposed method may result in a more accurate determination of the value of the cardiac parameter.

In some embodiments, the prediction value of each of the plurality of ultrasound image sets comprises the product of the view identification probabilities of the one or more ultrasound images of each of the plurality of ultrasound image sets. This product of probabilities may be a good indication of the probability that all images that make up a given image set have been identified correctly and its calculation and use may therefore allow a more reliable determination of the cardiac parameter.

In some embodiments, the cardiac parameter value comprises at least one of: an ejection fraction value; a segmental strain value; a global longitudinal strain value; and a longitudinal strain per view value. These parameters may be particularly useful in diagnosing disease and abnormalities in cardiovascular systems and therefore the proposed method may advantageously lead to greater effectiveness of diagnosis from ultrasound images.

According to another aspect of the present invention, there is provided a computer program comprising computer program code means adapted, when said computer program is run on a computer, to implement the method of any of the above discussed embodiments.

According to yet another aspect of the present invention, there is provided a system for determining a value of a cardiac parameter of a cardiovascular system based on a plurality of ultrasound images of the cardiovascular system, the system comprising: a processing arrangement configured to: process the plurality of ultrasound images with a view identification model to determine a view of each of the plurality of ultrasound images an associated view identification probability describing the likelihood the determined view of the ultrasound image is correct; determine, based on the plurality of ultrasound images and the determined view of each of the plurality of ultrasound images, a plurality of ultrasound image sets each comprising one or more ultrasound images of the plurality of ultrasound images; determine for each of the plurality of ultrasound image sets, based on the view identification probabilities of the one or more ultrasound images of each of the plurality of ultrasound image sets, a prediction value describing a predicted usefulness of the ultrasound image set for determining a value of the cardiac parameter of the cardiovascular system; and determine, based on the plurality of ultrasound image sets and the determined prediction value of each of the plurality of ultrasound images sets, a value of the cardiac parameter of the cardiovascular system.

The processing arrangement above may be integrated into an ultrasound image device or may be a separate device configured to communicate with ultrasound imaging systems known in the art.

Thus, there may be proposed concepts for providing a method of determining a value of a cardiac parameter of a cardiovascular system based on ultrasound images of the cardiovascular system which leverages the formation of different image sets based on the identified view of each of the plurality of ultrasound images.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for determining a value of a cardiac parameter of a cardiovascular system based on a plurality of ultrasound images of the cardiovascular system according to a proposed embodiment;
Fig. 2 is a simplified flow diagram of a method for determining a value of a left ventricular parameter of a cardiovascular system according to another proposed embodiment;
Fig. 3 is a simplified block diagram of a system for determining a value of a cardiac parameter of a cardiovascular system according to another aspect of the present invention; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should be understood that the same reference numerals are used throughout the Figures to indicate the same or similar parts.

Implementations in accordance with the present disclosure relate to various techniques, methods, schemes and/or solutions pertaining to a method for determining a cardiac parameter of a cardiovascular system. According to proposed concepts, a number of possible solutions may be implemented separately or jointly. That is, although these possible solutions may be described below separately, two or more of these possible solutions may be implemented in one combination or another.

Echocardiograms are commonly used by medical professionals to assess cardiovascular systems of patients. Typical echocardiogram examinations involve the acquisition of 50-70 ultrasound images from different scanning angles. The proposed method involves analyzing this plurality of ultrasound images to automatically identify the viewing direction of each of the images and determine based on this which of the images is suitable for the determination of a particular cardiac parameter. Although discussed mainly in the context of left ventricular parameters such as ejection fraction and global longitudinal strain, it will be understood that the concepts of the proposed method could be applied to the determination of any cardiac parameter. In particular, the method may be used to determine parameters of diastolic or systolic function of a cardiovascular system. These may comprise at least one of: fractional shortening; fractional area change; segmental wall motion; wall motion score index; ventricular and atrial end diastolic and end systolic volumes; end diastolic and end systolic diameters; cardiac wall thickness; and cardiac wall mass.

In summary, the proposed method for determining a value of a cardiac parameter comprises the following modules:
A. Ultrasound Image Data: a plurality of ultrasound images of the cardiovascular system of interest are acquired. These may typically be the plurality of images obtained in a single echocardiogram examination of a patient, however in some implementations of the proposed method these images may be obtained over the period of several different examinations. Each of the plurality of images depicts the cardiac system from a particular view direction.
B. View Identification Model: a model is used to automatically determine for each of the plurality of ultrasound images the view of the image. The view may describe the anatomical components visible in the image and/or the orientation or viewing angle of the image. The probability that the view of each image has been identified correctly is also generated by the view identification model.
C. Formation of Ultrasound Image Sets: the plurality of ultrasound images are organized into sets based on the determined view of each image. The sets can contain any number of ultrasound images greater than one and the formation of sets may be specific to the cardiac parameter that is determined. Certain view directions will be particularly useful for the evaluation of certain cardiac parameters.
D. Cardiac Parameter Determination: the view probabilities are used to predict the usefulness of each image set for the evaluation of the cardiac parameter. This is then used in the process of determining the value of the cardiac parameter to improve the efficiency and accuracy of the determination.

Various aspects of the proposed method may be implemented using machine learning algorithms. Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Purely by way of example, the machine learning model may employ a Convolutional Neural Network, because CNNs have been proven to be particularly successful at analyzing images, with a much lower error rate than other types of neural network. For the specific application of image inpainting problems: U-Nets, GANs, and Diffusion Probabilistic Models have also been shown to be particularly useful.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformations (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In the context of the present invention, the view identification model may comprise a machine learning model. In this case, the training input data entries correspond to real or simulated ultrasound images of cardiovascular systems. The training output data entries correspond to the views of these ultrasound images as manually annotated by a health care professional. In other words, the view identification model may be trained using a deep learning algorithm configured to receive an array of training inputs and respective known outputs, wherein a training input comprises an ultrasound image of a cardiovascular system and a respective known output comprises a known view of the ultrasound image. In this way, the view identification model may be trained to automatically determine the view of an ultrasound image from an echocardiogram examination. Machine learning models may also output a confidence level in the determination of the output which is used as the view identification probability in the present invention.

The determination of the value of the cardiac parameter may involve a machine learning model. In this instance, the training input data entries correspond to sets of real or simulated ultrasound images of a cardiovascular system, along with a prediction value for each set describing a predicted usefulness of each set of images for determining the value of the cardiac parameter. The training output data entries correspond to the value of the cardiac parameter of the cardiovascular system as manually annotated by a health care professional.

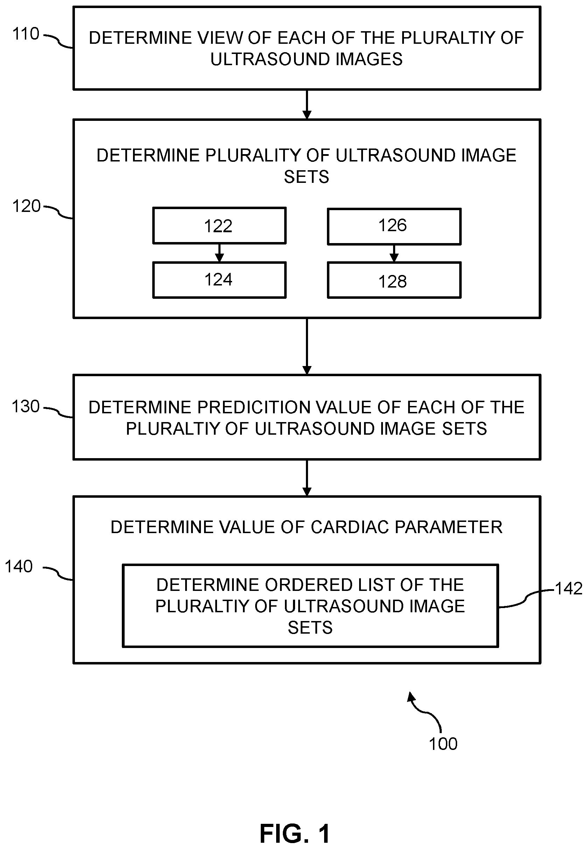

Referring now to Fig. 1, there is provided a simplified flow diagram of a method 100 for determining a cardiac parameter of a cardiovascular system based on a plurality of ultrasound images of the cardiovascular system according to a proposed embodiment. The exemplary method 100 comprises determining the value of a cardiac parameter comprising at least one of: an ejection fraction value; a segmental strain value; a longitudinal strain value; and a longitudinal strain per view value.

The method 100 commences with a step 110 comprising processing the plurality of ultrasound images with a view identification model to determine a view of each of the plurality of ultrasound images and an associated view identification probability describing the likelihood the determined view of the ultrasound images is correct. In this embodiment, the view identification model comprises a machine learning model trained to process an ultrasound image of a cardiovascular system and output the view of the image along with a view identification probability value describing the likelihood that the view of the ultrasound image has been identified correctly. In this exemplary embodiment the view of each of the plurality of ultrasound images comprises at least one of: parasternal long axis; parasternal short axis; and apical.

Once the view and an associated view identification probability have been determined for each of the plurality of ultrasound images, the method proceeds to step 120. The step 120 comprises determining, based on the plurality of ultrasound images and the determined view of each of the plurality of ultrasound images, a plurality of ultrasound image sets comprising one or more ultrasound images of the plurality of ultrasound images. The number of images contained within an image set and the views of the images that are selected for each image set may be predetermined based on the specific cardiac parameter that is being determined. Different cardiac parameters may be best evaluated by analyzing ultrasound images of different views; the proposed method may be advantageous in that it has the flexibility to be adapted to accurately determine a large variety of different cardiac parameters by altering the conditions by which images are selected for each image set. The plurality of ultrasound image sets are configured such that the images of any of the plurality of ultrasound image sets could be used in isolation to determine the value of the cardiac parameter.

In this exemplary embodiment, the step 120 comprises the sub-steps 122, 124, 126, and 128. Step 122 comprises comparing the view identification probability of each of the plurality of ultrasound images to a threshold probability value. The threshold probability in this exemplary method has a value in the range 0.7 to 1. Step 124 comprises disregarding a set of ultrasound images of the plurality of images having a view identification probability less than the threshold probability value. Thus, the method 100 involves filtering out a portion of the plurality of ultrasound images for which there is low confidence in the determined view value before the plurality of ultrasound images sets are formed.

In this exemplary embodiment, the plurality of ultrasound images comprise depth data, the depth data comprising a depth value of each of the plurality of ultrasound images. Step 126 then comprises comparing the depth value of each of the plurality of ultrasound images to a threshold depth value. Step 128 follows from step 126 and comprises disregarding a set of ultrasound images of the plurality of ultrasound images based on this comparison. In this exemplary embodiment, the threshold depth value is in the range 8 cm to 12 cm and disregarding the set of ultrasound images of the plurality of ultrasound images comprises disregarding the ultrasound images with depth values less than the threshold depth value. Thus, in the method 100 ultrasound images with too shallow depth values such that the images are unlikely to lead to an accurate estimation of the cardiac parameter, are filtered out before the plurality of sets of the ultrasound images are determined.

In other embodiments, the threshold depth value may be in the range 20 cm to 24 cm, and disregarding a set of ultrasound images of the plurality of ultrasound images may comprise disregarding the set of ultrasound images with depth values greater than the threshold depth value. Thus, some proposed methods may employ a maximum threshold depth value.

Once the plurality of ultrasound image sets have been determined, the method proceeds to step 130 which comprises determining for each of the plurality of ultrasound image sets, based on the view identification probabilities of the one or more ultrasound images of each of the plurality of ultrasound image sets, a prediction value describing a predicted usefulness of the ultrasound image set for determining a value of the cardiac parameter of the cardiovascular system. In exemplary method 100, the prediction value of each of the plurality of ultrasound image sets comprises the product of the view identification probabilities of the one or more images of the ultrasound image set. Thus, the prediction value may estimate the probability that the view of all ultrasound images of the ultrasound image set have been identified correctly.

Once the prediction value of each of the plurality of ultrasound image sets has been determined, the method proceeds to step 140 comprising determining, based on the plurality of ultrasound image sets and the determined prediction value of each of the plurality of ultrasound image sets, a value of the cardiac parameter of the cardiovascular system.

In this exemplary embodiment, the step 140 comprises the sub-step 142 which involves determining an ordered list of the plurality of ultrasound image sets based on the determined prediction value of each ultrasound image set. Thus, a list is generated starting with the ultrasound image set determined to be the most likely to be useful for determining the value of the cardiac parameter (i.e., in this instance the ultrasound image triplet for which it is most likely that all three images have correctly identified view). Determining the value of the cardiac parameter may then comprise working down the ordered list of ultrasound image sets and attempting to determine the value of the cardiac parameter based on the ultrasound image set at the top of the list. This may involve processing the ultrasound image with a cardiac parameter determination model. If the processing is unsuccessful and a value of the cardiac parameter cannot be determined, the ultrasound image set at the top of the ordered list is discarded and the cardiac parameter determination model is applied to the next ultrasound image set on the list. Thus, the proposed method may be advantageous in that only those images most likely to result in the determination of the value of the cardiac parameter are processed first, removing the need to process all the image sets to determine the value of cardiac parameter.

The cardiac parameter determination model may comprise a machine learning model. As discussed above, there are many known ways of training machine learning models to generate a required output from input data. In this instance, the cardiac parameter determination model may be trained using a deep learning algorithm configured to receive an array of training inputs and respective known outputs, wherein a training input comprises a set of ultrasound images of a cardiovascular system and a respective known output comprises a known value of a cardiac parameter of the cardiovascular system. In this way, the cardiac parameter determination model may be trained to automatically determine the value of a cardiac parameter from a selected set of ultrasound images.

Although in the method 100 the cardiac parameter comprises at least one of: an ejection fraction value; a segmental strain value; a longitudinal strain value; and a longitudinal strain per view value, in other embodiments the cardiac parameter could comprise any quantitative cardiac parameter that may be useful for diagnosis or assessment of a patient.

Similarly, other aspects of the method 100 may be implemented differently in alternative embodiments. For example, the step 120 need not comprise the filtering sub-steps 122, 124, 126, and 128. If it is known that all the ultrasound images of the plurality of ultrasound images are of high quality and that the view determination model has a high confidence level, these steps may be omitted in some embodiments. Further, the threshold probability value need not be in the range 0.7 to 1. The value of the threshold probability value may be selected based on known information about the quality of the plurality of ultrasound images or altered depending on the specific cardiac parameter being calculated. As well a minimum threshold depth value, a maximum threshold depth value may also be used. For example, the step 120 may comprise disregarding as set of ultrasound images of the plurality of ultrasound images with depth values greater than a threshold depth value in the rage 20 cm to 24 cm.

Alternatively, or in addition, further filtering steps may be performed in order to remove sets of ultrasound images that are unlikely to result in the accurate and reliable determination of the value of the cardiac parameter. For example, in instances wherein the plurality of ultrasound images comprise depth data comprising a depth value of each of the plurality of ultrasound images, and wherein each of the plurality of ultrasound image sets comprises two or more ultrasound images of the plurality of ultrasound images, determining the plurality of ultrasound image sets may comprise: determining a plurality of set candidates, each set candidate comprising two or more candidate images of the plurality of ultrasound images; comparing the depth values of the two or more candidate images of each of the plurality of set candidates; and disregarding a portion of set candidates of the plurality of set candidates based on this comparison to determine the plurality of ultrasound image sets. Thus, certain image sets of the number of possible image sets that could be formed may be disregarded if the different images of the set are at very different depths.

In other embodiments, the prediction value of an ultrasound image set may not be the product of the view identification probabilities of each image of the ultrasound image sets. Various algorithms and calculations may be performed based on the view identification probabilities of the images of an ultrasound image set to determine a prediction of the usefulness of the ultrasound image set for determining the value of the cardiac parameter. For example, the view identification probabilities may be compared to a predetermined threshold value and each of the plurality of ultrasound image sets categorized based on the number of images within the ultrasound image set for which the view identification probabilities are greater than the predetermined threshold value.

The method of the proposed invention does not require the formation of the ordered list of ultrasound image sets, as performed in step 142 of the method 100. Instead, a single image set of the plurality of ultrasound image sets may be selected, based on the determined prediction value of each of the plurality of ultrasound image sets, for processing with a cardiac parameter determination value.

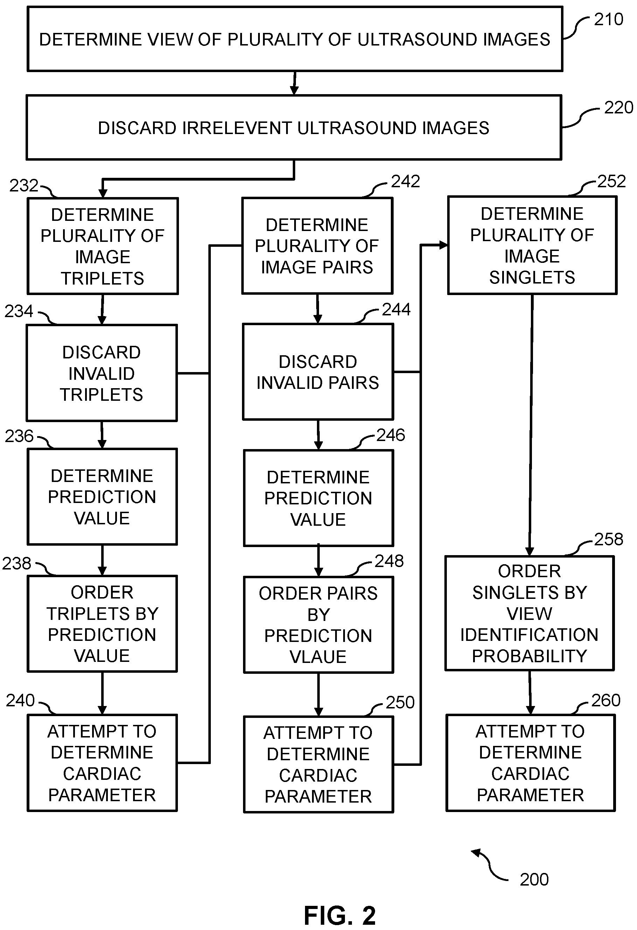

Referring now to Fig. 2, there is depicted a method 200 for determining the ejection fraction (EF) and global longitudinal strain (GLS) of a cardiovascular system according to a proposed embodiment.

Global left ventricular (LV) function evaluation is often performed during echocardiogram examinations. One of the frequently used parameters to evaluate LV function is the ejection fraction. The assessment of LV EF is important from management and prognosis of cardiology patients. For example, asymptomatic patients with ventricular dilation and reduced EF carry higher risk for mortality than the general population. In addition, quantitative EF measurement is essential for the management of patients with heart failure and influences therapeutic decisions.

Another commonly measured parameter is Longitudinal Strain, a value that expresses the shortening of the heart as a percentage number. Global Longitudinal Strain (GLS) is an accepted measurement used to characterize LV systolic function in clinical practice, in addition to the EF. It can identify subclinical LV dysfunction and it is routinely performed on chemotherapy patients since it allows early detection of impairment in the longitudinal function of the LV. A number of individual studies have shown GLS to be a prognostic marker in populations with known or suspected cardiovascular disease, including heart failure, valvular heart disease, cardiomyopathy, and ischemic heart disease. A method for determining accurate and reliable values of EF and GLS may therefore be particularly useful in the field of cardiology. A system that runs offline, performs rapid automated EF and GLS evaluation has the advantage of both standardization and time saving in the Echo lab by reducing examination time and subjectivity and enhancing patient care.

The method 200 commences with a step 210 comprising processing the plurality of ultrasound images of the cardiovascular system with a view identification model to determine a view of each of the plurality of ultrasound images and an associated view identification probability describing the likelihood the determined view of the ultrasound image is correct. In this embodiment, the view of each of the plurality of ultrasound images comprises one of: two-chamber apical (2ch); three-chamber apical (3ch); four-chamber apical (4ch); and other. All images that cannot be identified as having a 2ch, 3ch, or 4ch view are classified as "other". This may include images for which a view cannot be determined or images having a non-apical view direction. Thus, a model is used to identify which of these four view categories each image of the plurality of ultrasound images is most likely to fall under and assigning a view identification probability describing the confidence level in each of these categorizations. The 4ch, 3ch, and 2ch views are known to allow the most accurate determination of EF and GLS.

Once the view and associated view identification probability of each of the plurality of ultrasound images has been determined, the method proceeds to step 220 of the method 200 which comprises discarding irrelevant ultrasound images of the plurality of ultrasound images. This involves discarding all images for which the view has been categorized as "other" in the step 210 and discarding all images with a view identification probability less than a certain threshold value. Often images obtained during echocardiogram examinations further comprise depth data describing the depth of the plane depicted in each image. In this instance, step 220 may further comprise discarding images with depth values that indicate that it is unlikely that the value of the left ventricular parameter may be determined from the image, i.e. the plurality of ultrasound images may comprise depth data comprising a depth value of each of the plurality of ultrasound images and discarding irrelevant ultrasound images may comprise discarding a portion of the plurality of ultrasound images based on a comparison of the depth value of each of the plurality of ultrasound images to a threshold value.

Once the relevant images of the plurality of ultrasound images have been identified, the method proceeds to a step 232 of determining a plurality of ultrasound image triplets. Each ultrasound image triplet comprises: a first image of the plurality of ultrasound images determined to have a 2ch view; a second image of the plurality of ultrasound images determined to have a 3ch view; and a third image of the plurality of ultrasound images determined to have a 4ch view. Thus, the method involves determining, based on the view categorization carried out in step 210, all possible triplet sets of ultrasound images from the remaining plurality of ultrasound images of the cardiovascular system that comprise three images with different determined views.

In step 234, certain triplets of this plurality of ultrasound image triplets are discarded as being invalid. In this exemplary method, triplets are classified as invalid, and therefore discarded, based on a comparison of the depth values of the first, second, and third images of the plurality of ultrasound images. If the difference in the depth values of the first (3ch) image and the third (4ch) image is greater than 4 cm, or the difference in the depth values of the first (2ch) image and the second (3ch) image is greater than 6 cm, or the difference in the depth values of the second (3ch) image and the third (4ch) image is greater than 6 cm, the ultrasound image triplet is discarded. To achieve a reliable value of a quantitative cardiac image using more than one image view may require the image to be obtained at similar depths. Thus, this filtering step of the different possible triplets may result in a more reliable determination of the value of the EF and GLS. Although in this embodiment OR logic is employed to determine whether each of the plurality of ultrasound image triplets is valid based on a comparison on the difference of the depth values of the different images of the ultrasound image to predetermined threshold values, in some embodiments AND logic may be employed.

Once the valid ultrasound image triplets have been determined, the method proceeds to a step 236 of determining for each of the plurality of valid ultrasound image triplets, based on the view identification probabilities of the three ultrasound images of each of the plurality of ultrasound image triplets, a prediction value describing a predicted usefulness of the ultrasound image triplet for determining a value of the EF and GLS of the cardiovascular system. If no valid ultrasound image triplets are determined in the step 234, the method may proceed instead directly to step 242. In this exemplary embodiment, the prediction value is the product of the view identification probabilities of the first, second, and third images of each ultrasound image triplet.

The method then proceeds to a step 238 of determining an ordered list of the plurality of valid ultrasound images triplets based on the determined prediction value of each of the plurality of valid ultrasound images triplets. Thus, the first ultrasound image triplet of the ordered list may be the ultrasound image triplet with the highest prediction value and therefore the most likely to be useful for determining a value of the EF and GLS, and the last image triplet on the list is the ultrasound image triplet least likely to result in an accurate determination of the value of the ES and GLS of the cardiovascular system depicted in the plurality of ultrasound images.

The method 200 then proceeds to a step 240 comprising attempting to determine the value of the cardiac parameter based on the ordered list of the plurality of valid ultrasound image triplets. This involves, first processing the image at the top of the ordered list (i.e., the ultrasound image triplet with the highest prediction value) with a first cardiac parameter determination model configured to take an ultrasound image triplet of a cardiovascular system as input and output a value for the EF and GLS of the cardiovascular system. If this process in unsuccessful and no value for the EF and GLS is determined, the second image of the ordered list is processed with the cardiac parameter determination model in an attempt to determine the value of the EF and GLS. Thus, the method involves working down the ordered list of valid ultrasound image triplets in order of descending prediction value until either a value of the EF and GLS is determined or the end of the list is reached.

If no value of the EF and GLS can be determined from the valid ultrasound image triplets the method proceeds to the step 242, if a value is determined the method stops at this point. Step 242 comprises determining, based on the plurality of ultrasound images and their determined views and associated view identification probabilities, a plurality of ultrasound image pairs. Each ultrasound image pair comprises a first image of the plurality of ultrasound images determined to have a 2ch view, and a second image of the plurality of ultrasound images determined to have a 4ch view. Although the most accurate values of the EF and GLS are obtained from image triplets as described above, image pairs (and indeed single images) may also allow for EF and GLS values to be calculated.

The method then proceeds to a step 244 comprising discarding invalid ultrasound image pairs of the plurality of ultrasound image pairs. Similarly to step 234, determining whether a given ultrasound image pair is valid is achieved by comparing the depth values of the two images that form the pair and discarding ultrasound image pairs based on the comparison of the difference in the depth values of the 2ch and 4ch images to a predetermined threshold value. For each valid pair of ultrasound images that is determined, in step 246 a prediction value is calculated describing a prediction of the usefulness of the pair of images for determining the EF and GLS by taking the product of the view identification probabilities of the 2ch and 4ch images of each ultrasound image pair. If no valid image pairs can be formed, the method proceeds directly to step 252.

In step 248 the valid ultrasound image pairs are placed in an ordered list based on the calculated prediction value of each image pair. The ultrasound image pair with the highest prediction value is placed at the top of the list, and then the list continues in order of decreasing prediction value. The method then proceeds step 250 comprising attempting to determine a value of the EF and GLS based on the ordered list of image pairs. In a similar way to step 240, first, the first ultrasound image pair of the ordered list of image pairs is processed with a second cardiac parameter determination model. The second cardiac parameter determination model is configured to take an ultrasound image pair as an input and output a value of the EF and GLS. If processing the first ultrasound image pair of the ordered list of ultrasound image pairs does not result in a determination of the value of the EF and GLS for the cardiovascular system, the second ultrasound image pair of the ordered list of ultrasound image pairs is processed with the second cardiac parameter determination model and the first ultrasound image pair discarded. Thus, in a similar way to step 240, the ultrasound image pairs are processed in order of descending prediction value until either a value of the EF and GLS is successfully determined or there are no more ultrasound image pairs to process.

If the method has still not resulted in the determination of a value of the EF and GLS, the process of forming an ordered list is repeated for single images. Step 252 comprises determining a plurality of image singlets, each image singlet comprising a single ultrasound image of the plurality of ultrasound images. The prediction value of each image singlet is taken in this instance to be the view identification probability of the ultrasound image of the image single. Then, in step 258, the image singlets are then placed in an ordered list in order of decreasing view identification probability. This may involve forming three separate ordered lists, a first being an ordered list of images determined to have a 4ch view, the second being an ordered list of images determined to have a 3ch view, and the third being an ordered list of images of the plurality of ultrasound images determined to have a 2ch view.

Starting with the images with the highest view identification probability, in step 260, the plurality of ultrasound image singlets are processed with a third cardiac parameter determination model in order to attempt to determine a value of the EF and GLS. The third cardiac parameter determination model is configured to take single ultrasound images as inputs and output a value of the EF and GLS. Once a value has been determined, no further ultrasound image singlets are processed.

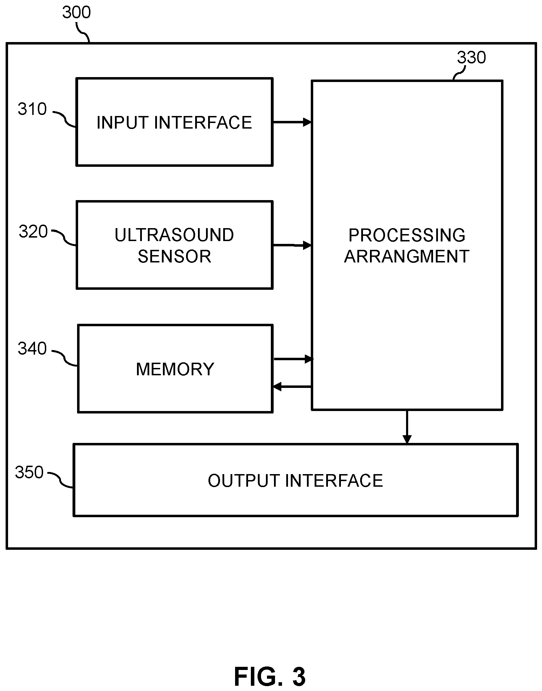

Referring now to Fig. 3 there is depicted a simplified block diagram of a system 300 for determining the value of a cardiac parameter of a cardiovascular system based on a plurality of ultrasound images of the cardiovascular system. The system 300 comprises an input interface 310, an ultrasound sensor 320, a processing arrangement 330, a memory 340, and an output interface 350.

The ultrasound sensor 320 is used to acquire a plurality of ultrasound images of a cardiovascular system of a patient during an echocardiogram examination. Once obtained, these are passed to the processing arrangement 330. The input interface 310 is configured to allow a clinician to select a specific cardiac parameter to be evaluated, this may be achieved via a touch sensitive screen for example. The selected cardiac parameter value is also passed to the processing arrangement 330.

The processing arrangement is configured to carry out the proposed method for determining the value of the cardiac parameter based on the plurality of ultrasound images. In detail the processing arrangement 330 is configured to: process the plurality of ultrasound images with a view identification model to determine a view of each of the plurality of ultrasound images and an associated view identification probability describing the likelihood the determined view of the ultrasound image is correct; determine, based on the plurality of ultrasound images and the determined views of each of the plurality of ultrasound images, a plurality of ultrasound image sets each comprising one or more ultrasound images of the plurality of ultrasound images determined to have a predetermined view; determine for each of the plurality of ultrasound image sets, based on the view identification probabilities of the one or more ultrasound images of each of the plurality of ultrasound image sets, a prediction value describing a predicted usefulness of the ultrasound image set for determining a value of the cardiac parameter of the cardiovascular system; and determine, based on the plurality of ultrasound image sets and the determined prediction value of each of the plurality of ultrasound image sets, a value of the cardiac parameter of the cardiovascular system.

The memory 340 may be associated with the processing arrangement 300. It may further store information on patient medical history. Once the value of the cardiac parameter has been determined it may be stored in the memory 340 alongside the patient's medical record. The value may also be output to the clinician carrying out the echocardiogram of the patient via the output interface 350. The output interface may be further configured to display to the clinician the set of ultrasound images used in the determination of the cardiac parameter of the cardiovascular system.

Although shown integrated into a single system 300, the ultrasound sensor, input interface, output interface and processing arrangement may each be found in separate devices. Thus, a processing arrangement of the present invention may integrate with existing ultrasound imaging systems.

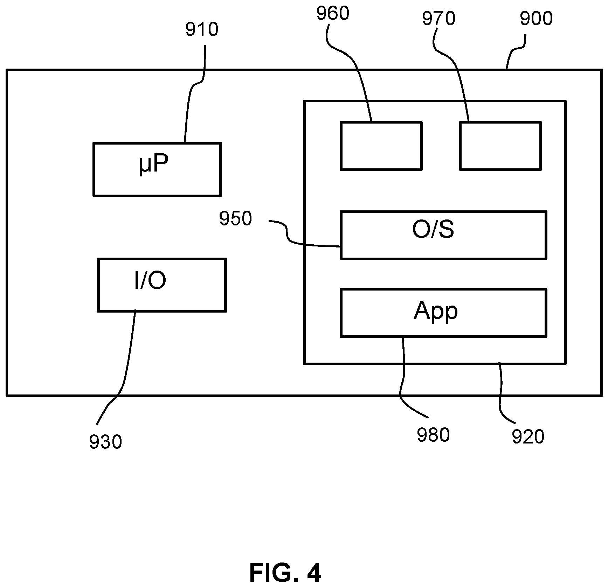

Fig. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A computer-implemented method (100) for determining a value of a cardiac parameter of a cardiovascular system based on a plurality of ultrasound images of the cardiovascular system, the method comprising:
processing (110) the plurality of ultrasound images with a view identification model to determine a view of each of the plurality of ultrasound images and an associated view identification probability describing the likelihood the determined view of the ultrasound image is correct;
determining (120), based on the plurality of ultrasound images and the determined views of each of the plurality of ultrasound images, a plurality of ultrasound image sets each comprising one or more ultrasound images of the plurality of ultrasound images;
determining (130) for each of the plurality of ultrasound image sets, based on the view identification probabilities of the one or more ultrasound images of each of the plurality of ultrasound image sets, a prediction value describing a predicted usefulness of the ultrasound image set for determining a value of the cardiac parameter of the cardiovascular system; and
determining (140), based on the plurality of ultrasound image sets and the determined prediction value of each of the plurality of ultrasound image sets, a value of the cardiac parameter of the cardiovascular system.

2. The computer-implemented method of claim 1, wherein the plurality of ultrasound image sets comprises a plurality of ultrasound image triplets, each of the plurality of ultrasound image triplets comprising:
a first image, being an ultrasound image of the plurality of ultrasound images determined to have a two-chamber apical (2ch) view;
a second image, being an ultrasound image of the plurality of ultrasound images determined to have a three-chamber apical (3ch) view; and
a third image, being an ultrasound image of the plurality of ultrasound images determined to have a four-chamber apical (4ch) view.

3. The computer-implemented method of claim 1, wherein the plurality of ultrasound image sets comprises a plurality of ultrasound image pairs, each of the plurality of ultrasound image pairs comprising:
a first image, being an ultrasound image of the plurality of ultrasound images determined to have a two-chamber apical (2ch) view; and
a second image, being an ultrasound image of the plurality of ultrasound images determined to have a four-chamber apical (4ch) view.

4. The computer-implemented method of claim 1, wherein determining the value of a cardiac parameter of the cardiovascular system comprises:
determining (142) an ordered list of the plurality of ultrasound image sets based on the determined prediction value of each ultrasound image set;
processing the first ultrasound image set of the ordered list with a cardiac parameter determination model; and
processing the second ultrasound image set of the ordered list with the cardiac parameter determination model if processing the first ultrasound image set of the ordered list does not result in a determination of the value of the cardiac parameter of the cardiovascular system.

5. The computer-implemented method of any preceding claim, determining the plurality of ultrasound image sets comprises:
comparing (122) the view identification probability of each of the plurality of ultrasound images to a threshold probability value, and
disregarding (124) a set of ultrasound images of the plurality of ultrasound images based on this comparison.

6. The computer-implemented method of claim 3, wherein the threshold probability value is in the range 0.7 to 1, and preferably wherein the threshold probability value is in the range 0.8 to 0.9.

7. The computer-implemented method of any preceding claim, wherein the plurality of ultrasound images comprise depth data comprising a depth value of each of the plurality of ultrasound images, and
wherein determining the plurality of ultrasound image sets comprises: comparing (126) the depth value of each of the plurality of ultrasound images to a threshold depth value; and
disregarding (128) a set of ultrasound images of the plurality of ultrasound images based on this comparison.

8. The computer-implemented method of claim 5, wherein the threshold depth value is in the range 8cm to 12 cm, and
wherein disregarding a set of ultrasound images of the plurality of ultrasound images comprises disregarding the set of ultrasound images with depth values less than the threshold depth value.

9. The computer-implemented method of any of claims 5 and 6, wherein the threshold depth value is in the range 20 cm to 24 cm,
and wherein disregarding a set of ultrasound images of the plurality of ultrasound images comprises disregarding the set of ultrasound images with depth values greater than the threshold depth value.

10. The computer-implemented method of any preceding claim, wherein the plurality of ultrasound images comprise depth data comprising a depth value of each of the plurality of ultrasound images, and
wherein each of the plurality of ultrasound image sets comprises two or more ultrasound images of the plurality of ultrasound images, and
wherein determining the plurality of ultrasound image sets comprises:
determining (232, 242) a plurality of set candidates, each set candidate comprising two or more candidate images of the plurality of ultrasound images determined to have a predetermined view;
comparing the depth values of the two or more candidate images of each of the plurality of set candidates; and
disregarding (234, 244) a portion of set candidates of the plurality of set candidates based on this comparison, to determine the plurality of ultrasound image sets.

11. The computer-implemented method of any preceding claim, wherein the prediction value of each of the plurality of ultrasound image sets comprises the product of the view identification probabilities of the one or more ultrasound images of each of the plurality of ultrasound image sets.

12. The computer-implemented method of any preceding claim, wherein cardiac parameter value is at least one of:
an ejection fraction value;
a segmental strain value;
a global longitudinal strain value; and
a longitudinal strain per view value.

13. A computer program comprising computer program code means adapted, when said computer program is run on a computer, to implement the method of any of claims 1-12.

14. A system (300) for determining a value of a cardiac parameter of a cardiovascular system based on a plurality of ultrasound images of the cardiovascular system, the system comprising:
a processing arrangement (330) configured to:
process the plurality of ultrasound images with a view identification model to determine a view of each of the plurality of ultrasound images and an associated view identification probability describing the likelihood the determined view of the ultrasound image is correct;
determine, based on the plurality of ultrasound images and the determined views of each of the plurality of ultrasound images, a plurality of ultrasound image sets each comprising one or more ultrasound images of the plurality of ultrasound images determined to have a predetermined view;
determine for each of the plurality of ultrasound image sets, based on the view identification probabilities of the one or more ultrasound images of each of the plurality of ultrasound image sets, a prediction value describing a predicted usefulness of the ultrasound image set for determining a value of the cardiac parameter of the cardiovascular system; and
determine, based on the plurality of ultrasound image sets and the determined prediction value of each of the plurality of ultrasound image sets, a value of the cardiac parameter of the cardiovascular system.

15. The system of claim 14, wherein the plurality of ultrasound image sets comprises a plurality of ultrasound image triplets, each of the plurality of ultrasound image triplets comprising:
a first image, being an ultrasound image of the plurality of ultrasound images determined to have a two-chamber apical (2ch) view;
a second image, being an ultrasound image of the plurality of ultrasound images determined to have a three-chamber apical (3ch) view; and
a third image, being an ultrasound image of the plurality of ultrasound images determined to have a four-chamber apical (4ch) view.
